# EUROPEAN PATENT SPECIFICATION

(11) **EP 3 728 610 B1**
(45) Date of publication and mention of the grant of the patent: **31.05.2023**
(21) Application number: 18826078.0
(22) Date of filing: 21.12.2018
(51) Int. Cl.: C12N 15/86, C07K 14/435, A61K 48/00

(54) **CONSTRUCTS COMPRISING NEURONAL VIABILITY FACTORS AND USES THEREOF**
KONSTRUKTE MIT NEURONALEN VIABILITÄTSFAKTOREN UND VERWENDUNGEN DAVON
CONSTRUCTIONS COMPRENANT DES FACTEURS DE VIABILITÉ NEURONALE ET LEURS UTILISATIONS

(30) Priority: 22.12.2017 EP 17306915
(43) Date of publication of application: 28.10.2020
(73) Proprietor: Sparingvision, 75008 Paris (FR); INSERM (Institut National de la Santé et de la Recherche Médicale), 75013 Paris (FR); SORBONNE UNIVERSITE, 75006 Paris (FR)
(72) Inventor: LEVEILLARD, Thierry, 94700 Maisons-Alfort (FR); AÏT-ALI MAAMRI, Najate, 75010 Paris (FR); BLOND, Frédéric, 94210 La Varenne Saint (FR); SAHEL, José-Alain, 75013 Paris (FR); PUEL, Géraldine, 77400 Thorigny sur Marne (FR); CLERIN, Emmanuelle, 91350 Grigny (FR)
(74) Representative: Plasseraud IP
(86) International application number: PCT/EP2018/086744
(87) International publication number: WO 2019/122403

(56) References cited:
- WO-A1-2016/185037
- WO-A1-2017/120294
- LEAH C. BYRNE ET AL: "Viral-mediated RdCVF and RdCVFL expression protects cone and rod photoreceptors in retinal degeneration", JOURNAL OF CLINICAL INVESTIGATION, vol. 125, no. 1, 21 November 2014 (2014-11-21), pages 105-116, XP055230095, US ISSN: 0021-9738, DOI: 10.1172/JCI65654
- Leah Caroline Thomas Byrne: "Engineered Adeno-Associated Viral Vectors for Gene Therapy in the Retina", UC Berkeley Electronic Theses and Dissertations, 1 January 2011 (2011-01-01), pages 1-81, XP055469692, Retrieved from the Internet: URL:https://escholarship.org/uc/item/3zz02 4j8 [retrieved on 2018-04-23]

## Description

### FIELD OF THE INVENTION

The present invention relates to retinal neurodegenerative disorders, and more particularly to a pharmaceutical composition for treating and/or preventing neurodegenerative disorders.

### BACKGROUND OF THE INVENTION

Neurodegenerative disorder encompasses a range of seriously debilitating conditions that are characterized by neuron degeneration.

Rod-cone dystrophies, such as retinitis pigmentosa (RP), are genetically heterogeneous retinal degenerative diseases characterized by the progressive death of rod photoreceptors followed by the consecutive loss of cones. RP is one of the most common forms of inherited retinal degeneration, affecting around 1:3,500 people worldwide (1). Mutations causing RP in over 63 distinct genes have been identified to date with a significant proportion of these mutations in rod-specific transcripts. RP patients initially present with loss of vision under dim-light conditions as a result of rod dysfunction, with relative preservation of macular cone-mediated vision. As the disease progresses, however, the primary loss of rods is followed by cone degeneration, and a deficit in corresponding cone-mediated vision. In modern society, in which much of the environment is artificially fit, and many activities rely on high acuity color vision, retention of cone-mediated sight in RP patients would lead to a significant improvement in quality of life.

The loss of cones in RP subsets caused by rod-specific mutations is not perfectly understood, although several mechanisms, which are not necessarily mutually exclusive, have been proposed. Some hypothesized mechanisms implicate a 'neighbor effect' whereby cone death is a consequence of the release of endotoxins from the degeneration of surrounding rods, or as a result of the loss of contact with rods, retinal pigment epithelium (RPE) or Müller glia. Alternatively, activation of Müller cells and the release of toxic molecules may play a role. Another hypothesis is that the quantities of oxygen or retinoids delivered to the photoreceptor layer by the RPE from the choroidal blood circulation are excessive and toxic as the metabolic load of rods is lost (2). Punzo et al. showed evidence that in murine models of retinal degeneration cones die in part as a result of starvation and nutritional imbalance, driven by the insulin/mammalian target of rapamycin pathway (3). Additionally, it has been suggested that the loss of a survival factor secreted by rods and required for cone survival may contribute to cone loss (4, 5). In agreement with the last hypothesis, transplanted healthy retinal tissue has been shown to support cone survival in areas distant from the grafted tissue in the *rdl* mouse (6, 7).

International patent application WO2008/148860A1 describes a family of trophic factors, called rod-derived cone viability factor (RdCVF) and RdCVF2 that are able to increase neuron survival and are useful for treating and/or preventing neurodegenerative disorders such as RP.

The rod-derived cone viability factor (RdCVF) was originally identified from a high-throughput method of screening cDNA libraries as a candidate molecule responsible for this rescue effect (4). Rods secrete RdCVF, and therefore, as rods die, the source of this paracrine factor is lost and RdCVF levels decrease. The loss of expression of RdCVF, and secreted factors like it, may therefore contribute to the secondary wave of cone degeneration observed in rod-cone dystrophies. RdCVF has been shown to mediate cone survival both in culture (8) and when injected subretinally in mouse and rat models of recessive and dominant forms of retinitis pigmentosa (4, 9). Disruption of *Nxnl1,* the gene encoding RdCVF, renders mouse photoreceptors increasingly susceptible to photoreceptor dysfunction and cone loss over time (10).

*Nxnl1* codes for two protein isoforms through differential splicing. The isoform mediating cone survival, RdCVF is a truncated thioredoxin-fold protein of its longer counterpart, RdCVFL, which includes a C-terminal extension conferring enzymatic thioloxidoreductase activity (11). RdCVFL, which contains all the amino acids of RdCVF, is encoded by exons 1 and 2 of the *Nxnl1* gene and is a member of the thioredoxin family (12). Thioredoxins have diverse functions, including maintaining the proper reducing environment in cells and participating in apoptotic pathways. These functions are accomplished via thioloxidoreductase reactions mediated by a conserved CXXC catalytic site within a thioredoxin fold (13).

Byrne *et al.* (32) have shown that the two isoforms of RdCVF have complementary functions. Systemic administration of an adeno-associated virus (AAV) encoding RdCVF improved cone function and delayed cone loss, while RdCVFL increased rhodopsin mRNA and reduced oxidative stress. RdCVFL prevents photo-oxidative damage to the rods (36).

International patent application WO 2016/185037 describes AAV vectors encoding both RdCVF and RdCVFL, in particular the AAV CT35, and the use of said vectors for treating pathologies such as retinitis pigmentosa.

A synergistic effect between RdCVF and RdCVFL has been demonstrated (34). On the one hand, RdCVF is produced and secreted by the retinal pigmented epithelium (RPE), protecting the cones by stimulating aerobic glycolysis through the RdCVF receptor at the cell surface of the cones by a non-cell autonomous mechanism(37). On the other hand, RdCVFL, protects the cones against oxidative damage in a cell autonomous manner, due to its thioloxidoreductase function.

The inventors have now observed that the production of such AAV vectors unexpectedly presents a problem of encapsidation of the AAV genome. This problem of incomplete packaging leads to a defect in the production of AAV particles with full genome.

This presents a limitation for the production of GMP-compliant AAV vectors for use in human therapy.

Thus, there is still a need for improved constructs for the expression of RdCVF and RdCVFL factors for treating retinal neurodegenerative disorders.

### SUMMARY OF THE INVENTION

The inventors have found that the production of a single AAV vector comprising a first nucleic acid encoding RdCVF and a second nucleic acid encoding RdCVFL could be subject to problems of incomplete packaging of the AAV single strand DNA within the viral capsid. This incomplete encapsidation leads to the production of an incomplete AAV genome, so a DNA of a smaller size.

The inventors have surprisingly discovered that this incomplete encapsidation was due to the presence of direct repeated sequences within the AAV genome and that limiting the length of nucleotides that are identical between the first and second expression cassettes, AAV can be fully packaged.

Thus, the present invention provides a solution to this problem, by providing AAV vectors comprising a first expression cassette comprising a first nucleic acid encoding RdCVF and a second expression cassette comprising a second nucleic acid encoding RdCVFL, in which production of AAV particles is optimized.

AAV according to the present application comprise a first and a second expression cassettes which display at most 70 contiguous identical nucleotides, in particular at most 60, 55, 54, 50, 40, 30, 20, 15, 10, 9 or 8 contiguous identical nucleotides.

The inventors have developed several alternative and/or cumulative solutions to the problem of direct repeated sequences between the first and second expression cassettes.

Thus, in one aspect, the disclosure relates to an adeno-associated vector (AAV) comprising:
- a first expression cassette comprising a first nucleic acid encoding RdCVF
   and
- a second expression cassette comprising a second nucleic acid encoding RdCVFL,
wherein said first and second expression cassettes display at most 70 contiguous identical nucleotides.

In another aspect, the disclosure relates to an adeno-associated vector (AAV) comprising:
- a first expression cassette comprising a first nucleic acid encoding RdCVF
   and
- a second expression cassette comprising a second nucleic acid encoding RdCVFL,
wherein said first and second expression cassettes display at most 70 contiguous identical nucleotides, for use in a method of treatment of a retinal neurodegenerative disorder,

The disclosure also relates to a method for treating a patient suffering from a retinal degenerative disease, said method being not part of the claimed invention, comprising the step consisting of administering to said patient a therapeutically effective amount of an adeno-associated vector (AAV) comprising:
- a first expression cassette comprising a first nucleic acid encoding RdCVF
   and
- a second expression cassette comprising a second nucleic acid encoding RdCVFL,
wherein said first and second expression cassettes display less than 200 contiguous identical nucleotides.

The disclosure also relates to the use of an inert human DNA sequence in an AAV construct which is not part of the claimed invention.

Thus, the present disclosure describes an AAV, not part of the claimed invention, which comprising a nucleic acid having the sequence set forth in SEQ ID NO: 10, wherein said nucleic acid having the sequence as set forth in SEQ ID NO: 10 is not present in an expression cassette.

### DETAILLED DESCRIPTION OF THE INVENTION

The invention is as defined in claims 1 -15.

The present invention relates to an adeno-associated vector (AAV) comprising:
- a first expression cassette comprising a first nucleic acid encoding RdCVF
   and
- a second expression cassette comprising a second nucleic acid encoding RdCVFL,
wherein said first and second expression cassettes display at most 70 contiguous identical nucleotides.

The fact that the first and the second expression cassettes display at most 70 contiguous identical nucleotides means that the first and the second expression cassettes have at most 70 contiguous identical nucleotides in common.

Typically, the first and second expression cassettes share at most 70 contiguous identical nucleotides, preferably at most 60, 55, 54, 50, 40, 30, 20, 15, 10, 9 or 8 contiguous identical nucleotides.

In a particular embodiment, the present invention relates to an adeno-associated vector (AAV) wherein said first and second expression cassettes display at most 54 contiguous identical nucleotides.

In a particular embodiment, the present invention relates to an adeno-associated vector (AAV) wherein said first and second expression cassettes display at most 9 contiguous identical nucleotides.

The present disclosure also describes an AAV, not part of the claimed invention, which comprises:
- a first expression cassette comprising a first nucleic acid encoding RdCVF
   and
- a second expression cassette comprising a second nucleic acid encoding RdCVFL,
wherein said first and second expression cassettes display at most 200 contiguous identical nucleotides, preferably at most 190, even more preferably at most 180, 170, 167, 165, 164, 160, 150, 140, 130, 120, 110, 100, 90, 80.

The present disclosure also describes further relates to an adeno-associated vector (AAV) comprising:
- a first expression cassette comprising a first nucleic acid encoding RdCVF
   and
- a second expression cassette comprising a second nucleic acid encoding RdCVFL,
wherein said first and second expression cassettes display at most 70 contiguous identical nucleotides, for use in a method of treatment of a retinal neurodegenerative disorder.

The present disclosure also describes an adeno-associated vector (AAV), not part of the claimed invention, which comprises:
- a first expression cassette comprising a first nucleic acid encoding RdCVF
   and
- a second expression cassette comprising a second nucleic acid encoding RdCVFL,
wherein said first and second expression cassettes display at most 200 contiguous identical nucleotides, for use in a method of treatment of a retinal neurodegenerative disorder.

The disclosure also relates to a method for treating a patient suffering from a retinal degenerative disease, not part of the claimed invention, which comprises a step consisting of administering to said patient a therapeutically effective amount of an adeno-associated vector (AAV) comprising:
- a first expression cassette comprising a first nucleic acid encoding RdCVF
   and
- a second expression cassette comprising a second nucleic acid encoding RdCVFL,
wherein said first and second expression cassettes display less than 200 contiguous identical nucleotides.

As used herein, the term Rod-derived Cone Viability Factor (RdCVF) refers to the protein encoded by the thioredoxin-like 6 (*TXNL6*) or Nucleoredoxin-like 1 (*NXNL1*) gene. It encompasses the RdCVF proteins of any animal species. Typically, the RdCVF proteins according to the present invention can be mammalian RdCVF proteins, including, but not limited to mice, rats, cats, dogs, non-human primates and human.

Unless otherwise specified, the term "RdCVF" refers to the short isoform of the *NXNL1* gene and "RdCVFL" or 'RdCVF-L" the long isoform of the *NXNL1* gene.

Typically, in mice, the short isoform (RdCVF) is a 109 amino-acid long protein references under Uniprot accession number Q91W38. The murine long isoform (RdCVFL) is a 217 amino-acid long protein referenced under Q8VC33.

In particular, the short isoform of RdCVF is the human short isoform of RdCVF (hRdCVF), having the following sequence:

         10         20         30         40         50
MASLFSGRIL IRNNSDQDEL DTEAEVSRRL ENRLVLLFFG AGACPQCQAF
         60         70         80         90        100
VPILKDFFVR LTDEFYVLRA AQLALVYVSQ DSTEEQQDLF LKDMPKKWLF
        109
LPFEDDLRR (SEQ ID NO: 1)

Accordingly, the first nucleic acid, encoding the short isoform of RdCVF can comprise the following human nucleic acid sequence:

ATGGCCTCCCTGTTCTCTGGCCGCATCCTGATCCGCAACAATAGCGACCAGGACGAGCTG
GATACGGAGGCTGAGGTCAGTCGCAGGCTGGAGAACCGGCTGGTGCTGCTGTTCTTTGGT
GCTGGGGCTTGTCCACAGTGCCAGGCCTTCGTGCCCATCCTCAAGGACTTCTTCGTGCGG
CTCACAGATGAGTTCTATGTACTGCGGGCGGCTCAGCTGGCCCTGGTGTACGTGTCCCAG
GACTCCACGGAGGAGCAGCAGGACCTGTTCCTCAAGGACATGCCAAAGAAATGGCTTTTC
CTGCCCTTTGAGGATGATCTGAGGAGGTGA (SEQ ID NO: 3)

Alternatively, the first nucleic acid can comprise a nucleic acid which differs from SEQ ID NO: 3 but encodes the same amino acid sequence.
Suitable nucleic acid sequences include, but are not limited to:
- polymorphisms of the cDNA encoding human RdCVF;
- combinations of polymorphisms (rare haplotypes) of the cDNA encoding human RdCVF. An example of rare haplotype cDNA is set forth as SEQ ID NO: 11;
- "optimized" sequences in which certain codons are replaced by codons that code for the same amino-acid. Suitable codon-optimized sequences encoding RdCVF include, but are not limited to, the sequence as set forth in SEQ ID NO: 12;
- homologous sequences. For instance, the inventors have found that the chimpanzee cDNA sequence encoding the short isoform of chimpanzee RdCVF can be used, since it encodes the same amino acid sequence as the human cDNA. The chimpanzee cDNA has the sequence as set forth in SEQ ID NO: 4.

In particular, the long isoform of the *NXNL1* gene is the human long isoform RdCVFL (hRdCVFL), having the sequence referenced under accession number Q96CM4 and set forth below:

10 20 30 40 50
MASLFSGRIL IRNNSDQDEL DTEAEVSRRL ENRLVLLFFG AGACPQCQAF
60 70 80 90 100
VPILKDFFVR LTDEFYVLRA AQLALVYVSQ DSTEEQQDLF LKDMPKKWLF
110 120 130 140 150
LPFEDDLRRD LGRQFSVERL PAVVVLKPDG DVLTRDGADE IQRLGTACFA
160 170 180 190 200
NWQEAAEVLD RNFQLPEDLE DQEPRSLTEC LRRHKYRVEK AARGGRDPGG
210
GGGEEGGAGG LF (SEQ ID NO: 2)

Accordingly, the second nucleic acid, encoding RdCVFL can comprise the following human nucleic acid sequence:

ATGGCCTCCCTGTTCTCTGGCCGCATCCTGATCCGCAACAATAGCGACCAGGACGAGCTG
GATACGGAGGCTGAGGTCAGTCGCAGGCTGGAGAACCGGCTGGTGCTGCTGTTCTTTGGT
GCTGGGGCTTGTCCACAGTGCCAGGCCTTCGTGCCCATCCTCAAGGACTTCTTCGTGCGG
CTCACAGATGAGTTCTATGTACTGCGGGCGGCTCAGCTGGCCCTGGTGTACGTGTCCCAG
GACTCCACGGAGGAGCAGCAGGACCTGTTCCTCAAGGACATGCCAAAGAAATGGCTTTTC
CTGCCCTTTGAGGATGATCTGAGGAGGGACCTCGGGCGCCAGTTCTCAGTGGAGCGCCTG

CCGGCGGTCGTGGTGCTCAAGCCGGACGGGGACGTGCTCACTCGCGACGGCGCCGACGAG
ATCCAGCGCCTGGGCACCGCCTGCTTCGCCAACTGGCAGGAGGCGGCCGAGGTGCTGGAC
CGCAACTTCCAGCTGCCAGAGGACCTGGAGGACCAGGAGCCACGGAGCCTCACCGAGTGC
CTGCGCCGCCACAAGTACCGCGTGGAAAAGGCGGCGCGAGGCGGGCGCGACCCCGGGGGA
GGGGGTGGGGAGGAGGGCGGGGCCGGGGGGCTGTTCTGA (SEQ ID NO: 5)

Alternatively, the second nucleic acid can comprise a nucleic acid which differs from SEQ ID NO:5 but encodes the same amino acid sequence.
Suitable nucleic acid sequences include, but are not limited to:
- polymorphisms of the cDNA encoding human RdCVF or combinations thereof;
- "optimized" sequences in which certain codons are replaced by codons that code for the same amino-acid; Suitable codon-optimized sequences encoding RdCVF include, but are not limited to, the sequence as set forth in SEQ ID NO:12;
- homologous sequences in other species.

The sequences of the RdCVF and RdCVFL proteins are described in Chalmel *et al.* 2007 (39) and in the international patent application WO2008/148860.

As used herein, the term "adeno-associated vector" or "AAV" has its general meaning in the art.

AAVs have been extensively described in the art as suitable vectors for gene delivery. Indeed, AAVs are non-pathogenic and display a broad range of tissue specificity, depending of their serotype. Typically, AAVs according to the present invention are AAVs that are able to target retinal cells.

Examples include, but are not limited to, AAV2, AAV8, AAV2/8, AAV2/5, AAV2/9, and AAV7m8.

In a particular embodiment of the invention, the AAV is a serotype AAV2/8.

In one embodiment, the AAV according to the present invention is obtained according to the method described in international patent application WO2012/158757.

Typically, the first and second nucleic acids, encoding respectively the short and long isoform of the *NXNL1* gene, are under the control of a promoter that allows the expression of said short and long isoform in the target cells.

Suitable promoters can be ubiquitous promoters, such as the CMV/CBA promoter. Suitable promoters can be promoters that enable the expression in the retina, preferably in retinal pigmented epithelial cells and photoreceptor cells.

In particular, the promoter allows gene expression in retinal pigmented epithelial cells.

In particular, the promoter allows gene expression in cone photoreceptors. A non-limiting example is the cone-opsin promoter.

Typically, the short isoform of the *NXNL1* gene is expressed at least by retinal pigmented epithelial cells and the long isoform is expressed at least by cone photoreceptor cells.

More particularly, different promoters are used to drive the expression of the short isoform and of the long isoform.

Typically, the short isoform of the *NXNL1* gene can be expressed under the control of the CMV/CBA promoter and the long isoform of the *NXNL1* gene can be expressed under the control of the cone-opsin promoter.

According to an embodiment, the first nucleic acid encoding RdCVF, in the AAV of the present invention, is under the control of an ubiquitous promoter, preferably the CMV/CBA promoter.

According to an embodiment, the second nucleic acid encoding RdCVFL, in the AAV of the present invention, is under the control of the cone-opsin promoter.

In particular, the two expression cassettes are inverted, with one expression cassette being 5' to 3' and the other expression cassette being 3' to 5'.

The inventors have found that this configuration was also suitable to avoid incomplete packaging.

In one embodiment of the invention, said adeno-associated vector (AAV) above described further comprises a stuffer sequence of SEQ ID NO: 10.

In a specific embodiment, the present invention relates an adeno-associated vector (AAV) comprising:
- a first expression cassette comprising a first nucleic acid comprising a codon-optimized cDNA encoding RdCVF, under the control of an ubiquitous promoter, preferably the CMV/CBA promotor,
   and
- a second expression cassette comprising a second nucleic acid comprising a codon-optimized cDNA encoding RdCVFL under the control of the cone-opsin promoter (*OPN1L*/*MW*)*.*

In one embodiment, the first nucleic acid comprising a codon-optimized cDNA encoding RdCVF has the sequence set forth in SEQ ID NO: 12.

In one embodiment, the second nucleic acid comprising a codon-optimized cDNA encoding RdCVFL has the sequence set forth in SEQ ID NO: 13.

In one embodiment the adeno-associated vector has the sequence as set forth in SEQ ID NO: 6 (corresponding to construct C03 of the Examples below).

In the context of the invention, the term "treating" or "treatment", as used herein, means reversing, alleviating, inhibiting the progress of, or preventing the disorder or condition to which such term applies, or one or more symptoms of such disorder or condition (e.g., retinal degenerative diseases).

The term "retinal degenerative diseases" encompasses all diseases associated with cone degeneration, retinal degenerative disease include but are not limited to Retinitis Pigmentosa, age-related macular degeneration, Bardet-Biedel syndrome, Bassen-Kornzweig syndrome, Best disease, choroidema, gyrate atrophy, Leber congenital amaurosis, Refsum disease, Stargardt disease or Usher syndrome.

In one embodiment of the invention, the retinal degenerative disease is Retinitis Pigmentosa.

According to the invention, the term "patient" or "patient in need thereof" is intended for a human or non-human mammal affected or likely to be affected with retinal degenerative diseases.

According to the present disclosure, a "therapeutically effective amount" of a composition is one which is sufficient to achieve a desired biological effect, in this case increasing the neuron viability. It is understood that the effective dosage will be dependent upon the age, sex, health, and weight of the recipient, kind of concurrent treatment, if any, frequency of treatment, and the nature of the effect desired. However, the preferred dosage can be tailored to the individual subject, as is understood and determinable by one of skill in the art, without undue experimentation.

The expression vector of the invention can be suitable for intraocular administration. In a particular embodiment of the invention, the expression vector is administered by sub-retinal injection.

In one aspect, the invention also relates to a pharmaceutical composition comprising an adeno-associated vector (AAV) and a pharmaceutically acceptable carrier, wherein said AAV comprises:
- a first expression cassette comprising a first nucleic acid encoding RdCVF
- a second expression cassette comprising a second nucleic acid encoding RdCVFL, wherein said first and second expression cassettes display at most 70 contiguous identical nucleotides.

The present disclosure also describes a pharmaceutical composition, not part of the claimed invention, which comprises an adeno-associated vector (AAV) and a pharmaceutically acceptable carrier, wherein said AAV comprises:
- a first expression cassette comprising a first nucleic acid encoding RdCVF
- a second expression cassette comprising a second nucleic acid encoding RdCVFL, wherein said first and second expression cassettes display at most 200 contiguous identical nucleotides.

The present disclosure also describes an AAV, not part of the claimed invention, which comprising a nucleic acid having the sequence set forth in SEQ ID NO:10, wherein said nucleic acid having the sequence as set forth in SEQ ID NO: 10 is not present in an expression cassette. This sequence SEQ ID NO: 10 has the role of stuffer DNA in the AAV. The role of a stuffer sequence is to increase the size of the vector in order to avoid that the proviral plasmid is encapsidated instead of the gene of interest.

Usually, stuffers corresponding to bacteriophage lambda DNA are used in AAV. However, the sequences of bacteriophage lambda most commonly used as stuffer contain open reading frames, the *nin* regions. Although there are considered as inert because of phylogenetic distance to Human, Cheng *et al.* (38) have demonstrated that such stuffer were not as inert as expected because the *nin* regions may have strong transcription activity. Thus, when AAV with such stuffer are administrated to a human patient, there is a risk of transcription of the bacteriophage lambda DNA.

Thus, it was important to develop a new inert stuffer. The inventors have found that the nucleic acid having the sequence as set forth in SEQ ID NO: 10 could unexpectedly be used as a "stuffer" DNA, in order to increase the size of the AAV constructs, in replacement of the bacteriophage lambda stuffer.. Said nucleic acid having the sequence as set forth in SEQ ID NO:10 has the great advantage of being inert because this sequence is a non-translated sequence, is not a miRNA target, is a non-telomeric sequence, it does not contain any origin of DNA replication and contains no nucleotides repeat. This sequence having any functional activity, there is not risk of transcription when it is used as a stuffer in an AAV.

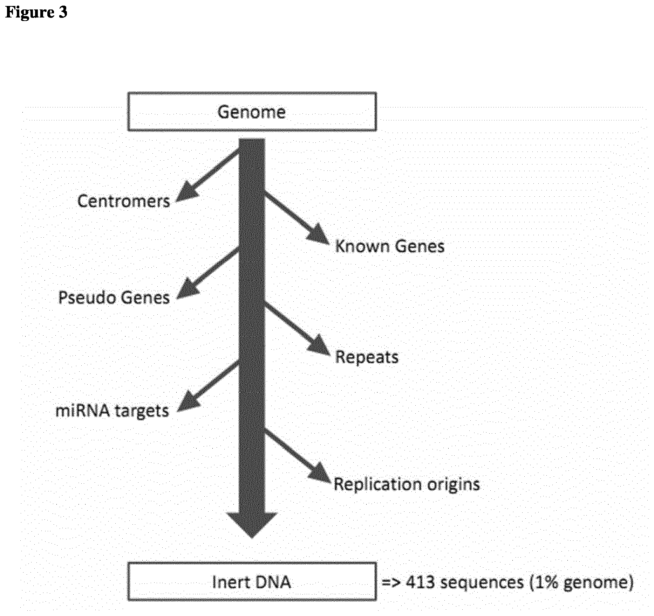

The sequence as set forth in SEQ ID NO: 10 was selected through a thorough process as described in Figure 3. It is a bioinformatic approach which consists of identifying in the human genome region that does not contains any elements as centromeres, genes, pseudo genes, replication origins, micro RNA targeted sequences or repeats. The sequence NO: 10 was selected among these loci.

The claimed invention will be further illustrated through the following examples and figures.

### FIGURES LEGENDS

**Figure 1****: Schematic representation of preferred constructs according to the invention:**
   Figure 1A represents the construct CT35, a comparative example (thus not a construct according to the present invention) disclosed in WO2016/185037. Figures 1B, 1C and 1D respectively represent constructs 3 (C03), 6 (C06) and 11 (C11) according to the invention.
**Figure 2****: Single strand DNA of AAV genome size analyzed by denaturing gel electrophoresis**
   The size of the single strand DNA of the AAV genome of different constructs was analyzed by gel electrophoresis under denaturing conditions:
   - Construct 3 (C03) which expresses both RdCVF and RdCVFL (7^{v7}. AAV2-CMV/CBA^{orig}-RdCVF-5'_1.7.*OPN1L*/*MW*-RdCVFL)
   - CT35 which expresses both RdCVF and RdCVFL (AAV2-CMV/CBA-RdCVF-CMV/CBA-RdCVFL) (SEQ ID NO: 15)
   - CT37 which only expresses RdCVF (AAV2-CMV/CBA-RdCVF + stuffer) (SEQ ID NO: 16).
**Figure 3****: Schematic representation of the selection process for an inert "stuffer" DNA**

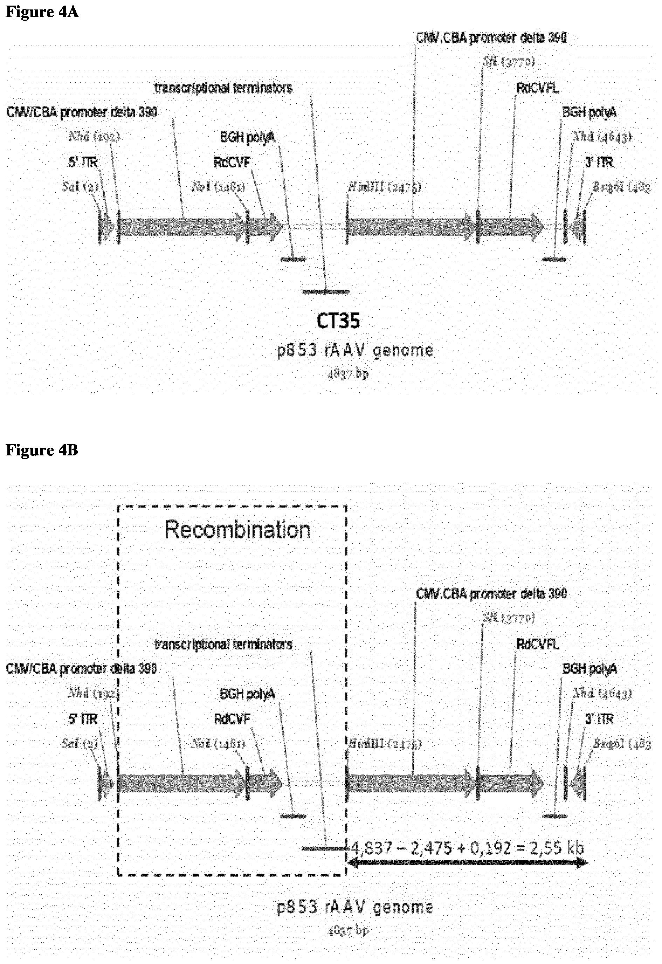

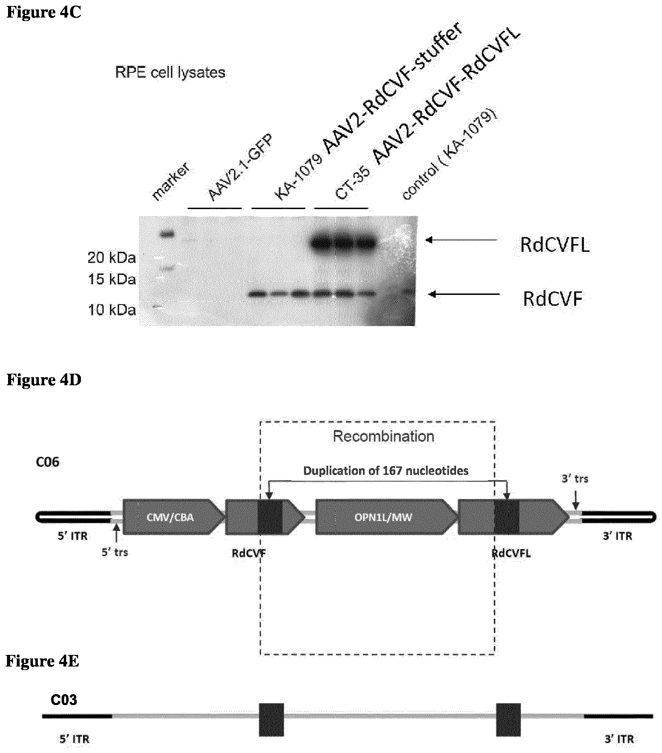

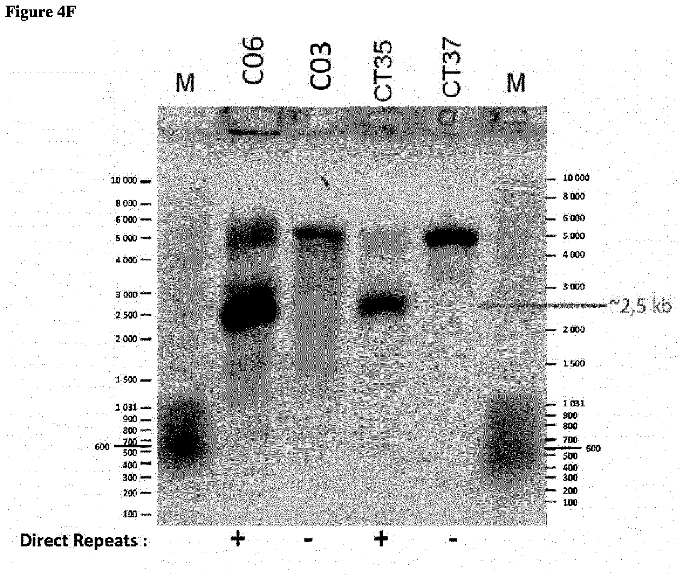

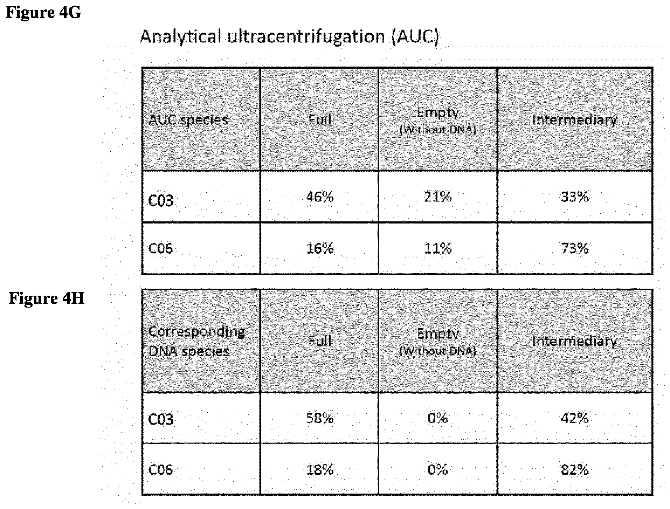

**Figure 4****: Packaging comparison**
**Figure 4A****:** Other representation of the AAV CT35.
**Figure 4B****:** Graphical simulation of a recombination between the two copies [CMV / CBA delta 390] which would produce an elimination of the cassette [CMV / CBA delta 390-RdCVF] or a recombination with the cassette [CMV / CBA delta 390-RdCVFL].
**Figure 4C****:** Transduction of CT35 (AAV-CMV / CBA-RdCVF_CMV / CBA-RdCVFL) in primary cells of porcine pigmented epithelium. Western blot analysis of RdCVF and RdCVFL using rabbit polyclonal anti-RdCVF antibodies (4).
**Figure 4D****:** Other representation of the AAV C06 and graphical simulation of a recombination. In this AAV, the first and the second expression cassettes share a direct repeat of 167 contiguous identical nucleotides.
**Figure 4E**: Other representation of the AAV C03.
**Figure 4F****:** Genome integrity analysis of encapsidated AAVs C06, C03, CT35 and CT37 (capsid proteins plus DNA).
**Figures 4G and 4H****:** Tables representing for C03 and C06 the percentage of capsids comprising a complete encapsidated AAV (full), the percentage of capsids comprising an incompletely encapsidated AAV (intermediate) and the percentage of capsids comprising no AAV (empty; without DNA). Table 4G shows detection results obtained for both capsid protein and DNA (AAV genome). Table 4H shows detection results obtained only for DNA.

### EXAMPLES

### Example 1

The following section provides non-limiting examples of suitable constructs according to the invention.

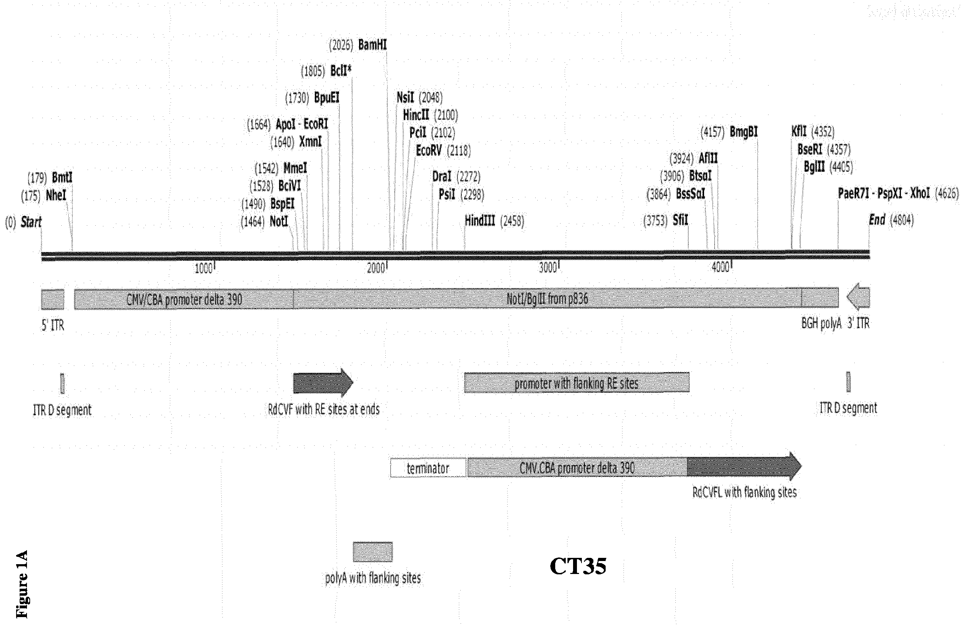

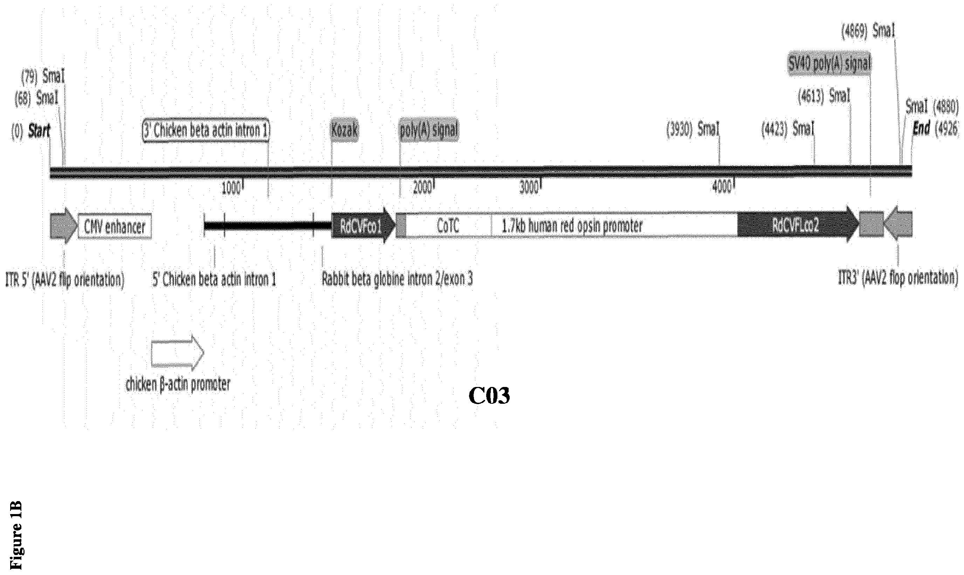

Construct 3 (C03): 7^{v7}. AAV2-CMV/CBA^{orig} -RdCVF-5'_1.7.*OPN1L*/*MW*-RdCVFL As shown on figure 1B, in this construct, the human RdCVF cDNA sequence was codon optimized (using a first optimization process v1) and placed under the ubiquitous promoter CMV/CBA. The human RdCVFL cDNA was also codon optimized (using a different optimization process v2) and was placed under the control of the cone-opsin promoter. The direct repeat shared between the first and the second expression cassette is 9 nucleotides long.

The AAV vector has the sequence as set forth in SEQ ID NO: 6.

### Construct 6 (C06):

### AAV2_CMV/CBA_orig_RdCVF_chimp_5p_1.7_OPN1LMW_RdCVFL

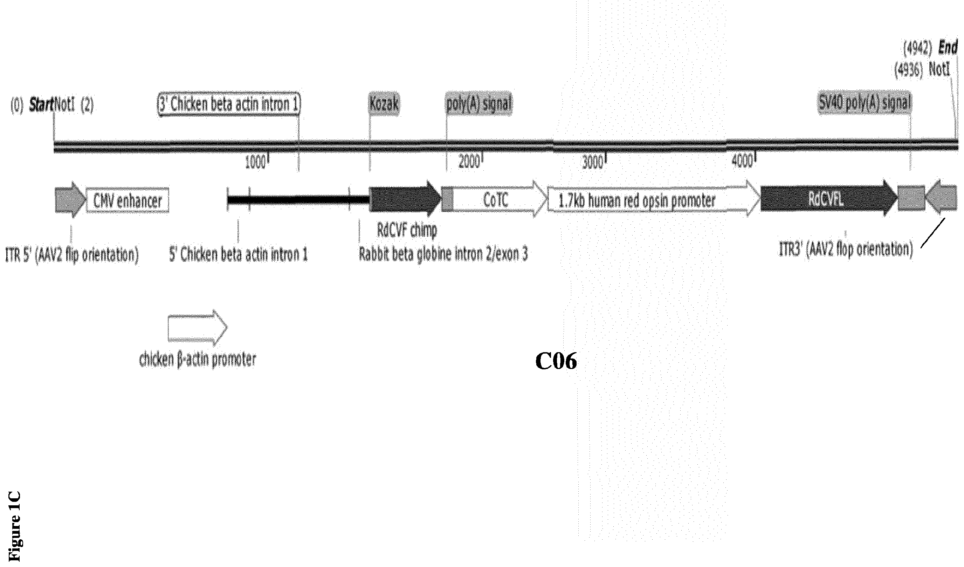

As shown on figures 1C and 4D, in this construct, the chimpanzee RdCVF cDNA sequence was used in the first expressed cassette and placed under the ubiquitous promoter CMV/CBA. The human RdCVFL cDNA was placed under the control of the cone-opsin promoter. The direct repeat shared between the first and the second expression cassette is 167 nucleotides long.

The AAV vector has the sequence as set forth in SEQ ID NO: 7.

### Construct 7 (C07):

### AA V2_rev_CMV/CBA_orig_RdCVF _chimp_5p_1.7 _OPNILMW _RdCVFL

This construct is similar to construct 6, except that the first expression cassette is placed in reverse orientation.

The AAV vector has the sequence as set forth in SEQ ID NO: 8.

### Construct 8 (C08):

### AAV2_CMV/CBA_orig_RdCVF_chimp_rev_5p_1.7_OPN1LMW_RdCVFL-hGH

This construct is similar to construct 6, except that the second expression cassette is placed in reverse orientation.

The AAV vector has the sequence as set forth in SEQ ID NO: 9.

### Construct 11 (C11):

### AAV2_CMV/CBA_orig_RdCVF_rare_haplotype_human_5p_1.7_OPN1LMW_RdCVF L

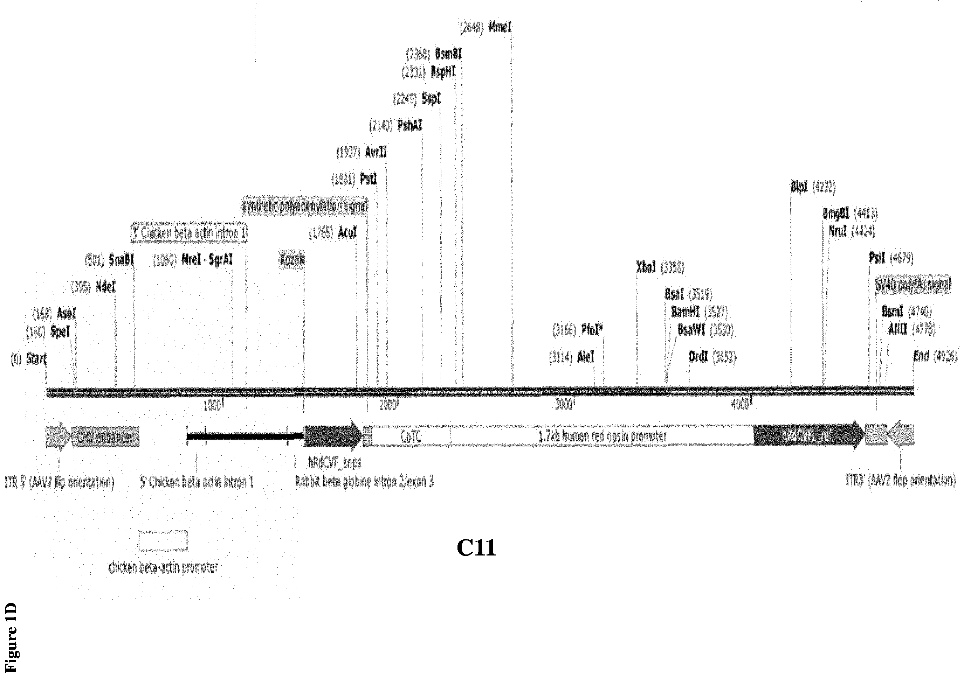

As shown on figure 1D, in this construct, the first expression cassette comprises a cDNA encoding human RdCVF that is a combination of polymorphisms (rare haplotype), under the ubiquitous promoter CMV/CBA. The second expression cassette comprises the human RdCVFL cDNA, under the control of the cone-opsin promoter. The direct repeat shared between the first and the second expression cassette is 54 nucleotides long.

The AAV vector has the sequence as set forth in SEQ ID NO: 14.

### Example 2: AAV constructs displaying long stretches of identical nucleotides are subject to incomplete packaging.

### Material and methods

### Production of viral vectors

AAV vectors carrying cDNA encoding mouse-RdCVF, RdCVFL or eGFP were produced by the plasmid co-transfection method (31). Recombinant AAV was purified by cesium chloride or iodixanol gradient ultracentrifugation. The viral eluent was buffer exchanged and concentrated with Amicon Ultra-15 Centrifugal Filter Units in PBS and titrated by quantitative PCR relative to a standard curve.

### Denaturing gel electrophoresis

The genomic DNA was extracted and subjected to denaturing gel electrophoresis. The size of the nucleic acids was compared to a DNA ladder.

### Results

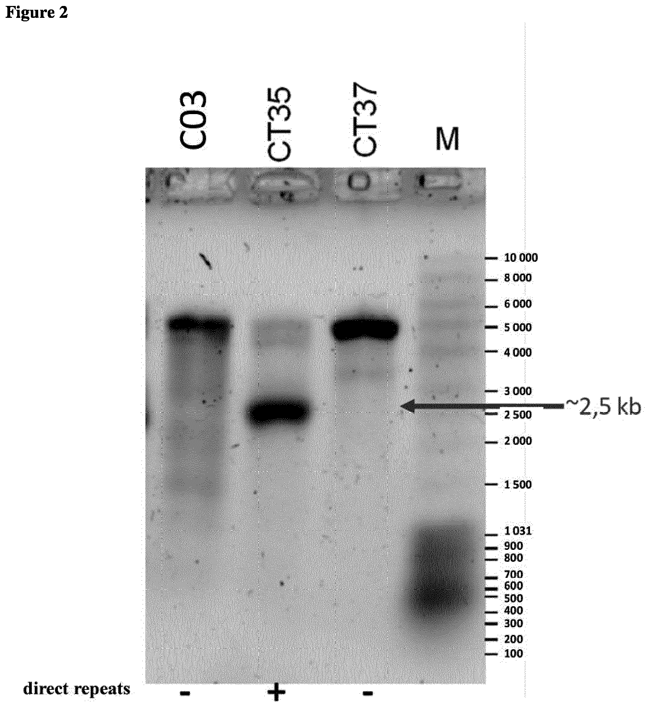

The figures 2 and 4F show that the construct CT37, disclosed in WO2016/185037 and thus not a construction according to the invention. This comparative example which only expresses RdCVF is subject to a complete packaging since its production results in DNA at the expected sizes of -5000 bp.

As shown at Figures 2 and 4F, CT35 is subject to incomplete packaging since its production results in abnormal AAV genome sizes instead of AAV genome of 4804 bp. Thus, it is well demonstrated that expressing a first nucleic acid encoding RdCVF and a second nucleic acid encoding RdCVFL in an AAV can lead to incomplete encapsidation of the AAV single strand DNA within the viral capsid.

In contrast, the construct C06 according to the invention, which expresses both RdCVF and RdCVFL and comprises a direct repeat of only 167 nucleotides long, show a band at the expected size of 4942 bp. This result demonstrates a complete encapsidation with the construct C06.

In the same way, the construct C03, which comprises a repeat of 9 contiguous identical nucleotides, shows a single band at the expected size of 4926 bp.

It is shown that decreasing the number of contiguous identical nucleotides shared between the two expression cassettes, allows to increase the proportion of complete encapsidation of an AAV comprising both a nucleic acid encoding RdCVF and a nucleic acid encoding RdCVFL.

Thus, the inventors have demonstrated that complete packaging is obtained when the first and second expression cassette do not contain more than 200 contiguous nucleic acids.

To further explore the phenomenon, analytical ultracentrifugation was used according to Burnham et al. (35) to compare the different constructs (Figures 4G and 4H). The results for C03 and C06 show that the extra band observed in the denaturing gel matches that of the high percentage of AAV particles with intermediary sedimentation coefficients, represent particles that are between full and empty, and thus corresponding to particles comprising an AAV incompletely packaged. In accordance with the results obtained in the denaturing gel electrophoresis, construct C06 shows full encapsidation of 18% and construct C03 yielded appropriate results in the analytical ultracentrifugation method, indicating a high percentage of full AAV particles (58%) (Figure 4H).

This confirms that there is an increase of the percentage of particles with integral genome when the number of contiguous identical nucleotides shared between the two expression cassettes is no superior to 200.

### Example 3: combination of RdCVF and RdCVFL results in a synergistic effect

The following constructs have been produced and introduced into an AAV2 vector.

The proviral plasmid p618 and its elements are described in international patent application published as WO2012158757A1 and in publication (33).

### 2xRdCVF: plasmid p857 and AAV CT39

P857/CT39 was designed to increase the level of expression of RdCVF as compared to CT37 (RdCVF-stuffer) to achieved sufficient cone protection in patients suffering from retinitis pigmentosa (RP).

### RdCVF-RdCVFL: plasmid p853 and AAV CT35

This vector is able to co-express the short and long isoform of RdCVF.

However, its production is subject to abnormal incomplete packaging events which limit its use as a therapeutic agent.

### Example 4: Selection of an inert DNA for replacing phage lambda stuffers

The inventors have developed a screening process for identifying a nucleic acid sequence which could be used as a safer alternative to the phage lambda stuffer sequences traditionally used in order to obtain AAV constructs having a sufficient size.

Screening of the entire human genome was performed in order to eliminate undesirable sequences such as centromers, known genes, pseudogenes, repeats, miRNA targets, replication origins. This inventive screening process resulted in the selection of SEQ ID NO: 10, which is an inert sequence from human chromosome 15.

### Example 5: Recombination analysis

Western blot analysis at Figure 4C shows the expression of RdCVF and RdCVFL using rabbit polyclonal anti-RdCVF antibodies. Both proteins are detected for the construct CT35, which demonstrates that CT35 is not subject to homologue recombination.

### REFERENCES

Throughout this application, various references describe the state of the art to which this invention pertains.
1. Buch H et al. Prevalence and causes of visual impairment and blindness among 9980 Scandinavian adults: the Copenhagen City Eye Study. Ophthalmology. 2004;111(1):53-61.
2. Bramall AN, Wright AF, Jacobson SG, McInnes RR. The genomic, biochemical, and cellular responses of the retina in inherited photoreceptor degenerations and prospects for the treatment of these disorders. Annu Rev Neurosci. 2010;33(1):441-472.
3. Punzo C, Kornacker K, Cepko CL. Stimulation of the insulin/mTOR pathway delays cone death in a mouse model of retinitis pigmentosa. Nat Neurosci. 2009;12(1):44-52.
4. Léveillard T et al. Identification and characterization of rod-derived cone viability factor. Nat Genet. 2004;36(7):755-759.
5. Mohand-Said S et al. Normal retina releases a diffusible factor stimulating cone survival in the retinal degeneration mouse. Proc Natl Acad Sci U S A. 1998;95(14):8357-8362.
6. Mohand-Said S et al. Photoreceptor transplants increase host cone survival in the retinal degeneration (rd) mouse. Ophthalmic Res. 1997;29(5):290-297.
7. Mohand-Said S, Hicks D, Dreyfus H, Sahel J-A. Selective transplantation of rods delays cone loss in a retinitis pigmentosa model. Arch Ophthalmol. 2000;118(6):807-811.
8. Wang XW, Tan BZ, Sun M, Ho B, Ding JL. Thioredoxin-like 6 protects retinal cell line from photooxidative damage by upregulating NF-kappaB activity. Free Radic Biol Med. 2008;45(3):336-344.
9. Yang Y et al. Functional Cone Rescue by RdCVF Protein in a Dominant Model of Retinitis Pigmentosa. Mol Ther. 2009;17(5):787-795.
10. Cronin T et al. The disruption of the rod-derived cone viability gene leads to photoreceptor dysfunction and susceptibility to oxidative stress. Cell Death Differ. 2010;17(7):1199-1210.
11. Brennan LA, Lee W, Kantorow M. TXNL6 is a novel oxidative stress-induced reducing system for methionine sulfoxide reductase a repair of α-crystallin and cytochrome C in the eye lens. PLoS ONE. [published online ahead of print: 2010]; doi:10.1371/journal.pone.0015421.g008
12. Funato Y, Miki H. Nucleoredoxin, a Novel Thioredoxin Family Member Involved in Cell Growth and Differentiation. Antioxid Redox Signal. 2007;9(8):1035-1058.
13. Lillig CH, Holmgren A. Thioredoxin and Related Molecules-From Biology to Health and Disease. Antioxid Redox Signal. 2007;9(1):25-47.
14. Barhoum R et al. Functional and structural modifications during retinal degeneration in the rdl0 mouse. Neuroscience. 2008;155(3):698-713.
15. Phillips MJ, Otteson DC, Sherry DM. Progression of neuronal and synaptic remodeling in the rd10mouse model of retinitis pigmentosa. J Comp Neurol. 2010;518(11):2071-2089.
16. Gargini C et al. Retinal organization in the retinal degeneration 10 (rd10) mutant mouse: A morphological and ERG study. J Comp Neurol. 2006;500(2):222-238.
17. Pang JJ et al. AAV-mediated gene therapy for retinal degeneration in the rd10 mouse containing a recessive PDEbeta mutation. Investigative Ophthalmology & Visual Science. 2008;49(10):4278-4283.
18. Pang JJ et al. Long-term retinal function and structure rescue using capsid mutant AAV8 vector in the rd10 mouse, a model of recessive retinitis pigmentosa. Mol Ther. 2011;19(2):234-242.
19. Komeima K, Rogers BS, Campochiaro PA. Antioxidants slow photoreceptor cell death in mouse models of retinitis pigmentosa. J Cell Physiol. 2007;213(3):809-815.
20. Dalkara D et al. In vivo-directed evolution of a new adeno-associated virus for therapeutic outer retinal gene delivery from the vitreous. Science Translational Medicine. 2013;5(189):189ra76.
21. Dalkara D et al. Enhanced gene delivery to the neonatal retina through systemic administration of tyrosine-mutated AAV9. Gene Ther. 2012;19(2): 176-181.
22. Cao W, Wen R, Li F, Lavail MM, Steinberg RH. Mechanical injury increases bFGF and CNTF mRNA expression in the mouse retina. Experimental Eye Research. 1997;65(2):241-248.
23. Hollander den AI, Black A, Bennett J, Cremers FPM. Lighting a candle in the dark: advances in genetics and gene therapy of recessive retinal dystrophies. J Clin Invest. 2010;120(9):3042-3053.
24. Maguire AM et al. Safety and Efficacy of Gene Transfer for Leber's Congenital Amaurosis. N Engl J Med. 2008;358(21):2240-2248.
25. Cideciyan AV et al. Human gene therapy for RPE65 isomerase deficiency activates the retinoid cycle of vision but with slow rod kinetics. Proceedings of the National Academy of Sciences. 2008;105(39):15112-15117.
26. Bainbridge JWB et al. Effect of Gene Therapy on Visual Function in Leber's Congenital Amaurosis. N Engl J Med. 2008;358(21):2231-2239.
27. Fridlich R et al. The Thioredoxin-like Protein Rod-derived Cone Viability Factor (RdCVFL) Interacts with TAU and Inhibits Its Phosphorylation in the Retina. Molecular & Cellular Proteomics. 2009;8(6):1206-1218.
28. Mingozzi F et al. CD8(+) T-cell responses to adeno-associated virus capsid in humans. Nat Med. 2007;13(4):419-422.
29. Manno CS et al. Successful transduction of liver in hemophilia by AAV-Factor IX and limitations imposed by the host immune response. Nat Med. 2006;12(3):342-347.
30. Jacobson SG et al. Gene therapy for leber congenital amaurosis caused by RPE65 mutations: safety and efficacy in 15 children and adults followed up to 3 years. Arch Ophthalmol. 2012;130(1):9-24.
31. Grieger JC, Choi VW, Samulski RJ. Production and characterization of adeno-associated viral vectors. Nat Protoc. 2006;1(3):1412-1428.
32. Byrne LC, Dalkara D, Luna G, Fisher SK, Clérin E, Sahel JA, Léveillard T, Flannery JG. Viral-mediated RdCVF and RdCVFL expression protects cone and rod photoreceptors in retinal degeneration. J Clin Invest. 2015 125(1): 105-16.
33. Vasireddy, V., Mills, J.A., Gaddameedi, R., Basner-Tschakarjan, E., Kohnke, M., Black, A.H., Alexandrov, K., Maguire, A.M., Chung, D.C., Mac, H., Sullivan, L., Gadue, P., Bennicelli, J.L., French, D.L., and Bennett, J. AAV-mediated gene therapy for choroideremia: Preclinical studies in personalized models. PLoS ONE 01/2013; 8(5):e61396.
34. Mei X., Chaffiol, A., Kole, C., Yang Y., Millet-Puel G., Clérin E., Ait-Ali N., Bennett, J., Dalkara D., Sahel JA, Duebel, J., Léveillard T., The thioredoxin encoded by the Rod-derived Cone Viability Factor gene protects cone photoreceptors against oxidative stress. Antioxid Redox Signal. 2016 May 12. [Epub ahead of print].
35. Burnham B, Nass S, Kong E, Mattingly M, Woodcock D, Song A, Wadsworth S, Cheng SH, Scaria A, O'Riordan CR: Analytical Ultracentrifugation as an Approach to Characterize Recombinant Adeno-Associated Viral Vectors. Human gene therapy methods 2015, 26(6):228-242.
36. Elachouri G, Lee-Rivera I, Clerin E, Argentini M, Fridlich R, Blond F, Ferracane V, Yang Y, Raffelsberger W, Wan J, Bennett J, Sahel JA, Zack DJ, Leveillard T: Thioredoxin rod-derived cone viability factor protects against photooxidative retinal damage. Free radical biology & medicine 2015, 81:22-29.
37. Ait-Ali N, Fridlich R, Millet-Puel G, Clerin E, Delalande F, Jaillard C, Blond F, Perrocheau L, Reichman S, Byrne LC, Ofivier-Bandini A, Bellalou J, Moyse E, Bouillaud F, Nicol X, Dalkara D, van Dorsselaer A, Sahel JA, Leveillard T: Rod-derived cone viability factor promotes cone survival by stimulating aerobic glycolysis. Cell 2015, 161(4):817-832.
38. Cheng SW, Court DL, Friedman DI: Transcription termination signals in the nin region of bacteriophage lambda: identification of Rho-dependent termination regions. Genetics 1995, 140(3):875-887.
39. Chalmel, F., Leveillard, T., Jaillard, C., Lardenois, A., Berdugo, N., Morel, E., Koehl, P., Lambrou, G., Holmgren, A., Sahel, J.A., and Poch, O. (2007). Rod-derived Cone Viability Factor-2 is a novel bifunctional-thioredoxin-like protein with therapeutic potential. BMC molecular biology 8, 74.

## Claims

1. An adeno-associated vector (AAV) comprising:
- a first expression cassette comprising a first nucleic acid encoding the short isoform of the Rod-derived Cone Viability Factor (RdCVF) and
- a second expression cassette comprising a second nucleic acid encoding the long isoform of the Rod-derived Cone Viability Factor (RdCVFL),
wherein said first and second expression cassettes display at most 70 contiguous identical nucleotides.

2. An AAV according to claim 1, wherein said first and second expression cassettes display at most 54 contiguous identical nucleotides.

3. An AAV according to claim 1, wherein said first and second expression cassettes display at most 9 contiguous identical nucleotides.

4. An AAV according to any one of claims 1 to 3, wherein the AAV is a serotype AAV2/8.

5. An AAV according to any one of claims 1 to 4, wherein the first nucleic acid encoding RdCVF is under the control of an ubiquitous promoter, preferably the CMV/CBA promoter.

6. An AAV according to any one of claims 1 to 5, wherein the second nucleic acid encoding RdCVFL is under the control of the cone-opsin promoter.

7. An AAV according to any one of claims 1 to 6, wherein the AAV has a nucleic acid sequence as set forth in the group consisting of SEQ ID NO: 6, SEQ ID NO: 8 SEQ ID NO: 9, and SEQ ID NO:14.

8. An AAV according to any one of claims 1 to 6, wherein the AAV has a nucleic acid sequence as set forth in SEQ ID NO: 6.

9. An AAV according to any one of claims 1 to 6, wherein the AAV further comprises a stuffer sequence of SEQ ID NO: 10.

10. An AAV according to claim 1 wherein the first nucleic acid comprises a codon-optimized cDNA encoding RdCVF as set forth in SED IQ NO:12, or wherein the second nucleic acid comprises a codon-optimized cDNA encoding RdCVFL as set forth in SED IQ NO:13.

11. A pharmaceutical composition comprising an AAV according to any one of claims 1 to 10 and a pharmaceutically acceptable carrier.

12. An AAV according to any one of claims 1 to 10, wherein said AAV is for use in a method of treatment of a retinal neurodegenerative disorder.

13. An AAV for use according to claim 12, wherein said retinal neurodegenerative disorder is chosen in the group consisting of: retinitis pigmentosa, age-related macular degeneration, Bardet-Biedel syndrome, Bassen- Komzweig syndrome, Best disease, choroidema, gyrate atrophy, Leber congenital amaurosis, Refsum disease, Stargardt disease and Usher syndrome.

14. An AAV for use according to claim 12, wherein said retinal neurodegenerative disorder is retinitis pigmentosa.

15. An AAV for use according to any one of claims 12 to 14, wherein said AAV is administered by sub-retinal injection.

## Patentansprüche

1. Adeno-assoziierter Vektor (AAV), umfassend:
- eine erste Expressionskassette, umfassend eine erste Nukleinsäure, die für die kurze Isoform des Stäbchen-abgeleiteten Zapfenviabilitätsfaktors (RdCVF) kodiert, und
- eine zweite Expressionskassette, umfassend eine zweite Nukleinsäure, die für die lange Isoform des Stäbchen-abgeleiteten Zapfenviabilitätsfaktors (RdCVFL) kodiert,
wobei die erste und die zweite Expressionskassette höchstens 70 zusammenhängende identische Nukleotide aufweisen.

2. AAV nach Anspruch 1, wobei die erste und die zweite Expressionskassette höchstens 54 zusammenhängende identische Nukleotide aufweisen.

3. AAV nach Anspruch 1, wobei die erste und die zweite Expressionskassette höchstens 9 zusammenhängende identische Nukleotide aufweisen.

4. AAV nach einem der Ansprüche 1 bis 3, wobei der AAV ein Serotyp AAV2/8 ist.

5. AAV nach einem der Ansprüche 1 bis 4, wobei die erste RdCVF kodierende Nukleinsäure unter der Kontrolle eines ubiquitären Promotors steht, vorzugsweise des CMV/CBA-Promotors.

6. AAV nach einem der Ansprüche 1 bis 5, wobei die zweite RdCVFL kodierende Nukleinsäure unter der Kontrolle des Zapfen-Opsin-Promotors steht.

7. AAV nach einem der Ansprüche 1 bis 6, wobei der AAV eine Nukleinsäuresequenz aufweist, wie sie in der Gruppe bestehend aus SEQ ID NO: 6, SEQ ID NO: 8, SEQ ID NO: 9 und SEQ ID NO: 14 dargestellt ist.

8. AAV nach einem der Ansprüche 1 bis 6, wobei der AVV eine Nukleinsäuresequenz wie in SEQ ID NO: 6 dargestellt aufweist.

9. AAV nach einem der Ansprüche 1 bis 6, wobei der AAV ferner eine Stuffer-Sequenz von SEQ ID NO: 10 umfasst.

10. AAV nach Anspruch 1, wobei die erste Nukleinsäure eine Codon-optimierte cDNA, die für RdCVF kodiert, umfasst, wie in SEQ ID NO:12 dargestellt, oder wobei die zweite Nukleinsäure eine Codon-optimierte cDNA, die für RdCVFL kodiert, umfasst, wie in SEQ ID NO: 13 dargestellt.

11. Pharmazeutische Zusammensetzung, umfassend einen AAV nach einem der Ansprüche 1 bis 10 und einen pharmazeutisch akzeptablen Träger.

12. AVV nach einem der Ansprüche 1 bis 10, wobei der AAV zur Verwendung in einem Verfahren zur Behandlung für eine retinale neurodegenerative Erkrankung ist.

13. AVV zur Verwendung nach Anspruch 12, wobei die retinale neurodegenerative Erkrankung ausgewählt ist aus der Gruppe bestehend aus: Retinitis Pigmentosa, altersbedingter Makuladegeneration, Bardet-Biedel-Syndrom, Bassen-Komzweig-Syndrom, Morbus Best, Choroidem, Gyratatrophie, Lebersche angeborene Amaurose, Refsum-Krankheit, Stargardt-Krankheit oder Usher-Syndrom.

14. AVV zur Verwendung nach Anspruch 12, wobei die retinale neurodegenerative Erkrankung Retinitis Pigmentosa ist.

15. AVV zur Verwendung nach einem der Ansprüche 12 bis 14, wobei der AVV durch sub-retinale Injektion verabreicht wird.

## Revendications

1. Vecteur adéno-associé (AAV) comprenant :
- une première cassette d'expression comprenant un premier acide nucléique codant pour l'isoforme courte du facteur de viabilité des cônes dérivé des bâtonnets (RdCVF) et
- une seconde cassette d'expression comprenant un second acide nucléique codant pour l'isoforme longue du facteur de viabilité des cônes dérivé des bâtonnets (RdCVFL),
dans lequel lesdites première et seconde cassettes d'expression affichent au plus 70 nucléotides identiques contigus.

2. AAV selon la revendication 1, dans lequel lesdites première et seconde cassettes d'expression affichent au plus 54 nucléotides identiques contigus.

3. AAV selon la revendication 1, dans lequel lesdites première et seconde cassettes d'expression affichent au plus 9 nucléotides identiques contigus.

4. AAV selon l'une quelconque des revendications 1 à 3, dans lequel l'AAV est un sérotype AAV2/8.

5. AAV selon l'une quelconque des revendications 1 à 4, dans lequel le premier acide nucléique codant pour RdCVF est sous le contrôle d'un promoteur ubiquitaire, de préférence le promoteur CMV/CBA.

6. AAV selon l'une quelconque des revendications 1 à 5, dans lequel le second acide nucléique codant pour RdCVFL est sous le contrôle du promoteur d'opsine des cônes.

7. AAV selon l'une quelconque des revendications 1 à 6, dans lequel l'AAV a une séquence d'acide nucléique telle qu'exposée dans le groupe constitué de SEQ ID NO : 6, SEQ ID NO : 8, SEQ ID NO : 9, et SEQ ID NO : 14.

8. AAV selon l'une quelconque des revendications 1 à 6, dans lequel l'AAV a une séquence d'acide nucléique telle qu'exposée dans SEQ ID NO : 6.

9. AAV selon l'une quelconque des revendications 1 à 6, dans lequel l'AAV comprend en outre une séquence de remplissage de SEQ ID NO : 10.

10. AAV selon la revendication 1 dans lequel le premier acide nucléique comprend un ADNc à codons optimisés codant pour RdCVF tel qu'exposé dans SEQ ID NO : 12, ou dans lequel le second acide nucléique comprend un ADNc à codons optimisés codant pour RdCVFL tel qu'exposé dans SEQ ID NO : 13.

11. Composition pharmaceutique comprenant un AAV selon l'une quelconque des revendications 1 à 10 et un vecteur pharmaceutiquement acceptable.

12. AAV selon l'une quelconque des revendications 1 à 10, dans lequel ledit AAV est destiné à être utilisé dans un procédé de traitement d'un trouble neurodégénératif de la rétine.

13. AAV pour son utilisation selon la revendication 12, dans lequel ledit trouble neurodégénératif de la rétine est sélectionné dans le groupe constitué de : une rétinite pigmentaire, une dégénérescence maculaire liée à l'âge, un syndrome de Bardet-Biedel, un syndrome de Bassen-Komzweig, une maladie de Best, une choroïdérémie, une atrophie gyrate, une amaurose congénitale de Leber, une maladie de Refsum, une maladie de Stargardt et un syndrome d'Usher.

14. AAV pour son utilisation selon la revendication 12, dans lequel ledit trouble neurodégénératif de la rétine est une rétinite pigmentaire.

15. AAV pour son utilisation selon l'une quelconque des revendications 12 à 14, dans lequel ledit AAV est administré par injection sous-rétinienne.
